# EUROPEAN PATENT APPLICATION

(11) **EP 4 071 480 A1**
(43) Date of publication of application: **12.10.2022**
(21) Application number: 21382305.7
(22) Date of filing: 09.04.2021
(51) Int. Cl.: G01N 33/68

(54) **CEREBROSPINAL FLUID BIOMARKER FOR ALZHEIMER'S DISEASE**

(71) Applicant: Fundació Ace. Institut Català de Neurociències Aplicades, 08028 Barcelona (ES); Universität zu Köln, 50923 Köln (DE)
(72) Inventor: Ruiz Laza, Agustín, 08028 Barcelona (ES); Orellana del Río, Adelina, 08028 Barcelona (ES); Martino Adami, Pamela Victoria, 50937 Köln (DE); Ramirez, Alfredo, 53757 Sankt Augustin (DE)
(74) Representative: Espiell Gomez, Ignacio

(57) **Abstract**

The present invention relates to a new biomarkers for the diagnosis of Alzheimer's disease (AD). More specifically, the present invention relates to the predictive detection of Alzheimer's disease (AD) in a mammal. The biomarkers, methods and kits provided enable prediction of progression of Mild Cognitive impairment (MCI) to AD by measurement of biomarkers in biological fluids that will provide an indication of whether a subject is likely to develop a neurological disease, such as Alzheimer's disease (AD).

## Description

### FIELD OF THE INVENTION

The present invention relates to the biological fluid biomarkers for Alzheimer's disease (AD). Particularly, the present invention relates to the MMP-10 biomarker for determining the progression of MCI (Mild Cognitive Impairment) to AD. More particularly, the present invention relates to improving the prognostic value in MCI patients by including MMP-10 to the existing markers for AD.

### DESCRIPTION OF THE PRIOR ART

There are more than 70 conditions that produce clinical dementia in humans, of which Alzheimer's Disease (AD) is the most common. Alzheimer's disease (AD) is a neurodegenerative disease characterized by memory loss and results in a progressive loss of cognitive function and dementia affecting one in eight people by the time they reach 65 years of age. Several pathways contribute to the pathology leading to neurodegeneration, cognitive decline, and finally dementia. Information on individual pathological pathways is provided by biomarkers. Currently established biomarkers mirror the histopathological hallmarks of the disease following the amyloid cascade hypothesis, i.e. cerebral amyloid deposition and tau-related neurodegeneration.

With regards to biomarkers for AD, the proteins amyloid beta and tau are probably the most well-characterized. Research has shown that cerebrospinal fluid ("CSF") samples from AD patients contain higher than normal amounts of tau, which is released as neurons degenerate, and lower than normal amounts of beta amyloid, presumably because it is trapped in the brain in the form of amyloid plaques. Because these biomarkers are released into CSF, a lumbar puncture (or "spinal tap") is required to obtain a sample for testing. Additionally, the current diagnostic [A/T/(N)] (amyloid/tau pathology/neurodegeneration) classification scheme of AD pathology considers decreased cerebrospinal fluid (CSF) levels of amyloid β-42 (Aβ42) reflecting amyloid pathology (A), increased phosphorylated-tau (P-tau) concentration as a marker of AD-specific tau pathology (T), and total tau (T-tau) level indicating neuronal injury (N). However, results from amyloid-specific therapies suggest that additional pathways are involved in AD and its progression that are not covered by existing biomarkers.

AD has a long asymptomatic phase that offers a substantial time-window for intervention. Utilizing this window of opportunity will require early diagnostic and prognostic biomarkers to detect AD pathology at pre-dementia stages, thus allowing identification of patients who will most probably progress to AD dementia (ADD) and benefit from specific disease-modifying therapies.

From a clinical perspective, defining a good toolbox of biomarkers for AD covering different pathogenic pathways will derive in better etiological diagnosis, especially at the early stages of the disease. In addition, it will have the potential to improve the prediction of disease progression, as well as to follow the effects of novel therapeutic approaches in clinical trials. While most research has been focused on defining biomarkers able to describe a specific clinical picture cross-sectionally, less research has been devoted to defining whether biomarkers are also able to predict disease progression along with the AD clinical staging, including preclinical subjective cognitive decline (SCD) stage, prodromal mild cognitive impairment (MCI) stage, and finally clinical dementia stage

Therefore, diagnostic and prognostic biomarkers are required to detect AD pathology, either in prodromal or preclinical disease stages or in delineating additional pathological pathways related to neurobiological processes which, potentially, modulate progression along the clinical stages e.g. biological fluid biomarkers are needed.

### SUMMARY OF INVENTION

The present invention provides a biomarker for predicting the risk of Alzheimer's disease (AD) in a mammal, or for aiding in the diagnosis of AD and related disorders, comprising metalloproteinase 10 (MMP-10).

In one of the embodiments, the invention discloses a method for predicting the risk of Alzheimer's disease (AD) in a mammal, or for aiding in the diagnosis of AD and related disorders, the method comprising determining the levels of matrix metalloproteinase 10 (MMP-10), wherein said amount is indicative of the presence, risk, or progression of said disease

In another embodiment, the MMP-10 is used in combination with the existing classification scheme of AD pathology biomarkers i.e. [A/T/(N)] (amyloid/tau pathology/neurodegeneration).

The mammal is preferably a human and an amyloid positive/tau negative mild cognitive impairment (MCI) patient. The biomarkers are obtained from a biological fluid sample wherein the biological fluid sample is cerebral spinal fluid (CSF), blood, serum, or plasma.

The MMP-10 levels predict the disease progression at the pre-dementia stage of Alzheimer's disease (AD) and further improves the prognostic value in amyloid positive/tau negative mild cognitive impairment (MCI) patients.

The MMP-10 predicts the disease progression from mild cognitive impairment (MCI) to overt dementia.

The MMP-10 levels predict the progression of MCI to Alzheimer's Disease Dementia (ADD).

In another embodiment, the present invention is directed towards a kit comprising the biomarker comprising MMP-10, wherein the kit is used to determine the presence or risk of AD in a mammal, or for aiding in the diagnosis and AD and related disorders.

### DETAILED DESCRIPTION

In the following description, for purposes of explanation, numerous specific details are outlined to provide a thorough understanding of the present disclosure. It will be apparent, however, to one skilled in the art that the present disclosure can be practiced without these specific details.

Reference in this specification to "one embodiment" or "an embodiment" means that a particular feature, structure, or characteristic described in connection with the embodiment is included in at least one embodiment of the present disclosure. The appearance of the phrase "in one embodiment" in various places in the specification are not necessarily all referring to the same embodiment, nor are separate or alternative embodiments mutually exclusive of other embodiments. Further, the terms "a" and "an" herein do not denote a limitation of quantity, but rather denote the presence of at least one of the referenced items. Moreover, various features are described which may be exhibited by some embodiments and not by others.

The embodiments are described herein for illustrative purposes and are subject to many variations. It is understood that various omissions and substitutions of equivalents are contemplated as circumstances may suggest or render expedient but are intended to cover the application or implementation without departing from the spirit or the scope of the present disclosure.

The following terms used in the specification have the following meanings:
The term "predicting" refers to making a finding with a notably enhanced likelihood of developing AD or related diseases.

The term "mammal", is preferably a human. Mammals include, but are not limited to, humans, primates, farm animals, sport animals, rodents and pets. It is further considered that the term "mammal", "subject", "patient" can be used interchangeably to refer to the same test subject being examined or analysed for the presence of biomarkers and evaluated for determining the status of a neurological disease, such as AD.

The terms methods for "aiding diagnosis" or "assisting in diagnosis" both refer to methods that assist in making a clinical determination regarding the presence or progression of the AD or related diseases, and may or may not be conclusive with respect to the definitive diagnosis.

The terms "Mild cognitive impairment patient," or "MCI patient," refer to an individual who has been diagnosed or suffering from MCI or has been given a probable diagnosis of MCI. Further, the stable mild cognitive impairment (sMCI) refers to MCI with no progression to ADD during the observational time. The progressive mild cognitive impairment (pMCI) refers to MCI with progression to ADD at any follow-up examination.

The term "biological fluid sample" encompasses a variety of fluid sample types obtained from an individual and can be used in a diagnostic assay. The definition encompasses, e.g., blood, cerebral spinal fluid (CSF), serum, or plasma and other liquid samples of biological origin.

Three different biomarkers in CSF have been particularly well documented: neuronal thread protein, tau (total; T-tau and various phosphorylated forms; P-tau), and derivatives of amyloid precursor protein (APP) including Aβ40 and Aβ42. The 2018 guidelines of the NIA-AA Research Framework grouped the AD biomarkers into those related to β-amyloid deposition, pathologic tau, and neurodegeneration [A/T/(N)]. However, the research framework stated that novel biomarkers describing different pathologic processes could be added to the [A/T/(N)] scheme proposed in 2016 with the notation [A/T/X/(N)], where X is the new biomarker. The present invention shows that adding MMP-10 to the markers already present in the scheme, with the notation "[A/T/M/(N)]", improved considerably the prognostic value in MCI patients with abnormal Aβ42, but normal P-tau181 and T-tau. The MCI patients with abnormal Aβ42 and MMP-10, but normal P-tau181 and T-tau, progressed to dementia with a median of one year, while MCI patients with abnormal Aβ42, but normal P-tau181, MMP-10, and T-tau, progressed to dementia with a median of two years.

The present invention discloses MMP-10 as a biomarker that predicts the disease progression of MCI to ADD, targeting pathways beyond those covered by the current AD core biomarkers. The MCI subjects with increased CSF level of MMP-10 shows a higher probability of progressing to ADD.

In one of the embodiments, the present invention discloses a biomarker comprising matrix metalloproteinase 10 (MMP-10) biomarker to determine the presence or risk of AD in a mammal, or for aiding in the diagnosis and AD and related disorders.

In another embodiment, the present invention discloses a scheme of biomarkers [A/T/M/(N)] comprising matrix metalloproteinase 10 (MMP-10) in combination with (amyloid/tau pathology/neurodegeneration) classification scheme of AD pathology biomarkers.

MMP-10 (also known as stromelysin-2) is a zinc-dependent enzyme that belongs to the ubiquitous superfamily of matrix metalloproteinases (MMPs), with more than 20 family members which are predominantly secreted. In the extracellular space, this family of proteases degrades extracellular matrix proteins participating in tissue remodeling and contributes to the cleavage of cell adhesion molecules, cytokines, and growth factors. Many MMPs are expressed in the brain by astrocytes and microglia, though several are expressed only under very specific physiological and/or pathological conditions. In AD, research has shown that MMPs expression is induced by Aβ and they are also able to degrade Aβ. In the case of MMP-10, cross-sectional studies showed that the CSF level of MMP-10 is increased in both AD and MCI patients who showed Aβ42 pathology. The present invention reflects the previous observations in MCI, and also supports the independent role of MMP-10 as a biomarker for disease progression, and not just a downstream epiphenomenon linked to amyloid pathology.

In another embodiment, MMP-10 alone influences the risk of progression to dementia of the Alzheimer-type similarly as Aβ42. The invention discloses that MMP-10 poses a risk that is independent of amyloid pathology. The CSF levels of P-tau¹⁸¹ and T-tau are highly correlated, suggesting that these two biomarkers provide information on overlapping pathways in AD. Conversely, MMP-10 exhibits a moderate correlation with the core AD biomarkers, adding thereby an additional value.

In a further embodiment, the present invention discloses a method for predicting the progression of mild cognitive impairment (MCI) to Alzheimer's disease dementia (ADD) in a mammal, wherein the method comprises determining the cerebrospinal fluid (CSF) levels of matrix metalloproteinase 10 (MMP-10).

The invention is applicable to any mammal, preferably to human beings.

In another embodiment, the invention discloses that MMP-10 protein level tags pathways related to ageing, since the CSF level of MMP-10 increases steadily with age, and MMP-10 and age protein correlation profiles are highly associated. The correlation between MMP-10 and age indicates that the potential amyloid-independent role of MMP-10 in dementia and its added value in our [A/T/M/(N)] classification is related to ageing. In fact, MMP-10 expression was found increased in a model of dermal ageing, and it was also increased following tissue damage within the context of age-related diseases, such as diabetes and atherosclerosis. Like in these chronic diseases, age is by far the largest risk factor for dementia and ADD. Besides, MMP-10 levels have been also associated with Parkinson's disease progression, another age-related neurodegenerative disease, further supporting the general effect of MMP-10 in neurodegeneration.

Ageing is a complex process involving several biological changes that lead to chronic cellular stress and sterile tissue inflammation. During this process, some cells become senescent and start producing a unique secretory profile, termed the senescence-associated secretory phenotype (SASP), which includes an increase in inflammatory mediators, such as IL-6, TNF-β, TGFβ family ligands, MIF, YKL-40, and also proteases. Like other cell types, microglia and astrocytes, the main source of MMP-10 in the brain, become senescent as a result of ageing and neurodegeneration. Interestingly, SASP has been observed in the CSF of ADD patients, and senescent astrocytes express increased levels of MMP-3 and MMP-10 as part of their SASP.

Besides its role in ageing, neuroinflammation triggered by microglia and astrocytes plays a central role in the pathogenesis of AD. While microglia constitute a first line of defense showing activation and fast recruitment to sites of damage to phagocytose dead cells and debris, astrocytes are activated following microglial reaction. Activation of microglia leads, among other things, to changes in the MMPs expression profile, including an increase of MMP-10 mRNA, and activated astrocytes release inflammatory mediators that signal back to microglia and can recruit infiltrating hematogenous cells including macrophages. During the progression of AD, synaptic dysfunction and amyloid pathology might activate microglia and astrocytes, leading to a high neuroinflammation status that in turn enhances neuronal damage. Hence, senescence and neurodegeneration might act in concert enhancing neuroinflammation leading to worsened patient prognosis, with MMP-10 serving as a marker of this interplay.

Other features and advantages of the present invention will become apparent on reading the following examples, which are to be considered as illustrative and not limiting.

### DESCRIPTION OF FIGURES

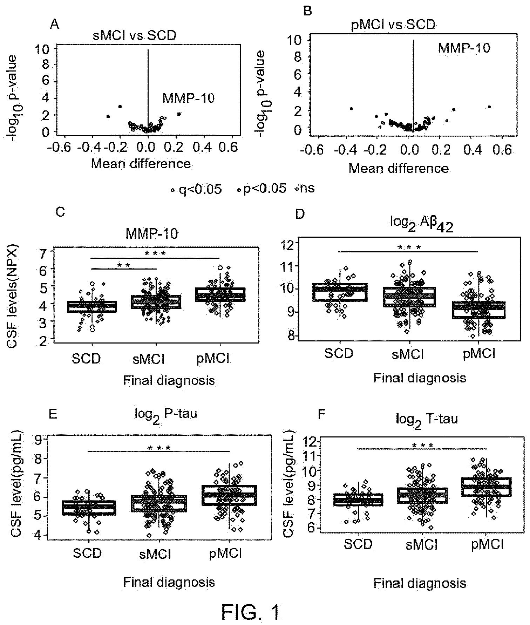

FIG.1: CSF levels of MMP-10 and core AD biomarkers in relation to diagnosis in FACE cohort. Volcano plots show the pattern of differential expression (mean difference) of the 137 ProSeek^{®} Multiplex panels proteins in the CSF of stable MCI (A) and progressive MCI (B) vs SCD subjects. Light blue, significant false discovery rate (FDR)-corrected p value (q value); orange, nominally significant (p value); violet, not significant. Box plots show the CSF level of MMP-10 (C), log2-transformed Aβ42 (D), log2-transformed P-tau181 (E), and log2-transformed T-tau (F) in SCD, stable MCI and progressive MCI diagnostic groups. SCD, subjective cognitive decline; sMCI, stable mild cognitive impairment; pMCI, progressive mild cognitive impairment. **p < 0 01, ***p < 0 001.

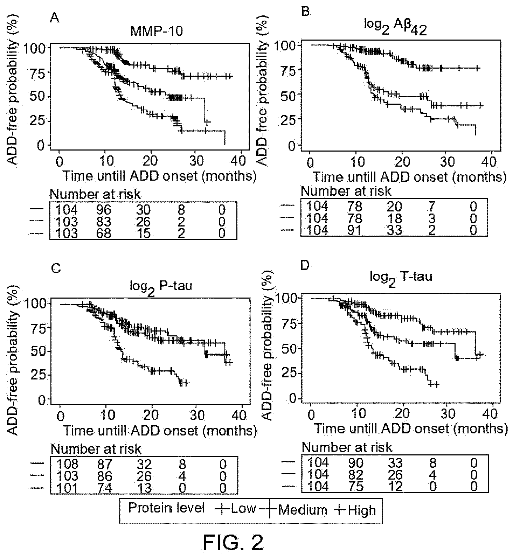

FIG.2: Association between CSF levels of MMP-10 and core AD biomarkers with progression from MCI to ADD in FACE cohort. Kaplan-Meier curves show the association of the CSF levels of MMP-10 (A), log2-transformed Aβ42 (B), log2-transformed P-tau181 (C), and log2-transformed T-tau (D) of MCI subjects with the probability of not progressing to ADD. Protein levels were divided into tertiles with similar number of subjects. Yellow, first fertile (lowest protein level); blue, second tertile (medium protein level); gray, third tertile (highest protein level). Each vertical line represents a censored event. ADD, dementia of Alzheimer's disease type.

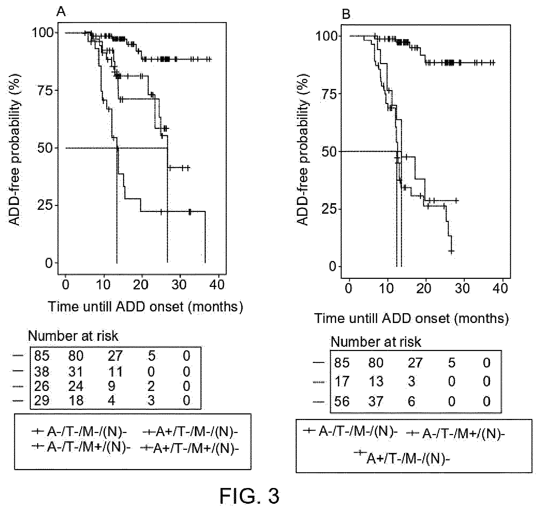

FIG.3: Risk stratification with A/T/M/(N) scheme for MCI subjects in FACE cohort. Kaplan-Meier curves show the association of A-/T-/M-/(N)-, A-/T-/M+/(N)-, A+/T-/M-/(N)- and A+/T-/M+/(N)- (A) or A-/T-/M-/(N)-, A+/T+/M-/(N)+ and A+/T+/M+/(N)+ (B) categories with the probability of not progressing to ADD. Dotted lines represent the median survival. Each vertical line represents a censored event. ADD, dementia of Alzheimer's disease type.

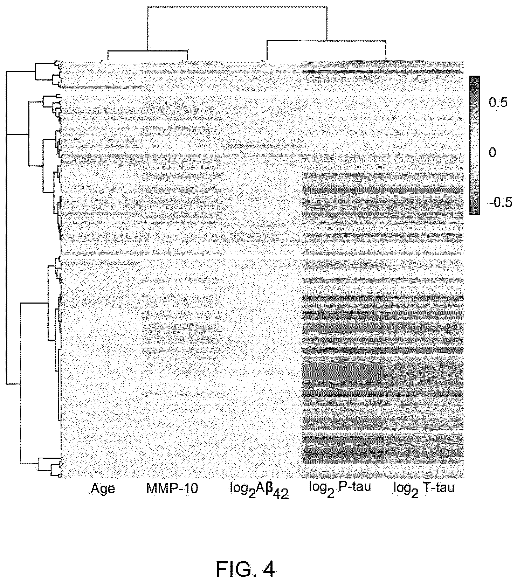

FIG.4: Correlation clustering of ageing, MMP-10, Aβ42, P-tau and T-tau with ProSeek^{®} Multiplex panels proteins in FACE cohort. Heat map shows the hierarchical clustering among ProSeek^{®} Multiplex panels proteins (rows) and age, MMP-10, Aβ42, P-tau and T-tau (columns). Scale indicates Pearson correlation distance (1 - Pearson correlation coefficient).

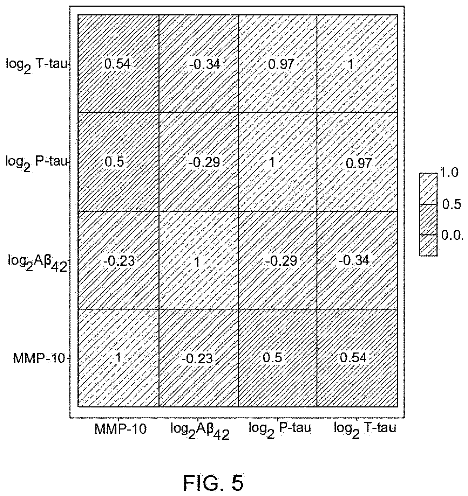

FIG.5: Correlation among the CSF levels of MMP-10 and core AD biomarkers of SCD and MCI subjects from FACE cohort. The correlation matrix shows the degree of correlation among MMP-10 (z-transformed), Aβ42 (z log2-transformed), P-tau (z log2-transformed), and T-tau (z log2-transformed), being gray a negative correlation, and blue a positive correlation. Pearson correlation coefficients (p) are also provided for each pair.

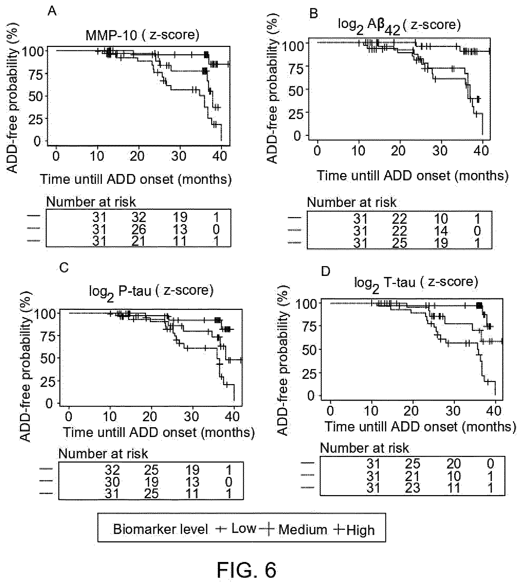

FIG.6: Association between CSF levels of MMP-10 and core AD biomarkers with progression from MCI to ADD in DCN cohort. Kaplan-Meier curves show the association of the CSF levels of MMP-10 (A), log2-transformed Aβ42 (B), log2-transformed P-tau (C), and log2-transformed T-tau (D) of MCI subjects with the probability of not progressing to ADD. Protein levels were divided into tertiles with similar number of subjects. Yellow, first tertile (lowest protein level); blue, second tertile (medium protein level); gray, third tertile (highest protein level). Each vertical line represents a censored event. ADD, dementia of Alzheimer's disease type.

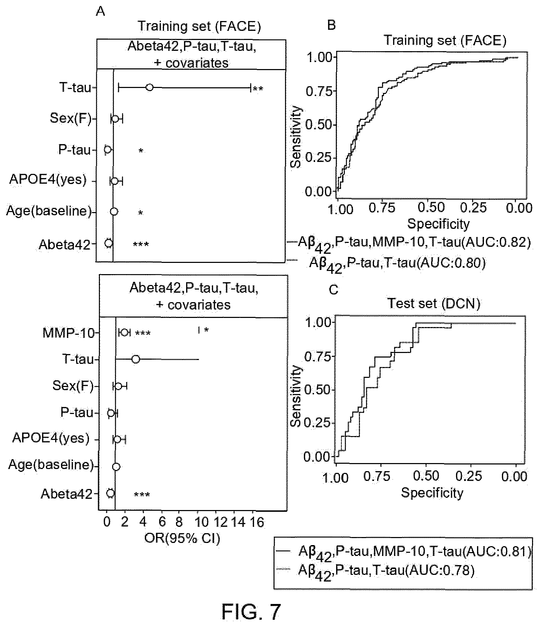

FIG.7: Predictive value of CSF MMP-10 and core AD biomarkers for progression from MCI to ADD. Forest plots show the odds-ratio (95% confidence interval) of each predictor in a model including the z log2-transformed CSF levels of Aβ42, P-tau181, and T-tau, plus age, sex and presence of APOE-ε4 allele (A, upper panel), or in a second model including the z-transformed CSF level of MMP-10 (A, lower panel) for MCI subjects from FACE cohort (training set). Receiver operating characteristic (ROC) curves with their respective areas under the curve (AUC) display the diagnostic ability of each model in FACE (training set) (B) and in DCN cohort (test set) (C). Absence of the APOE-ε4 allele and being male were used as reference variables. *p < 0 05, **p < 0 01, ***p < 0 001.

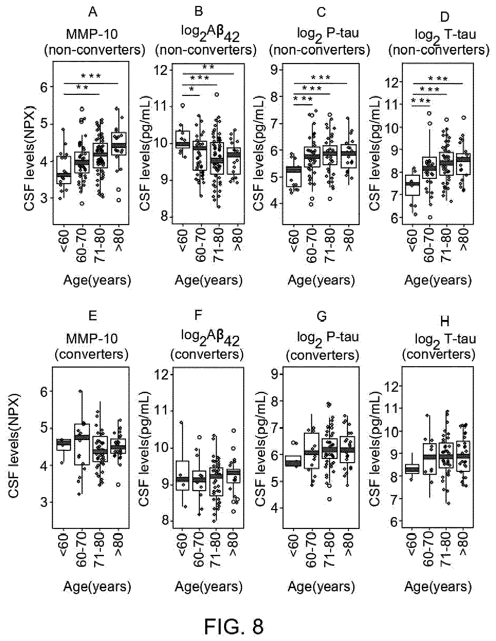

FIG.8: CSF levels of MMP-10 and core AD biomarkers in relation with age and conversion status in FACE cohort. Box plots show the CSF level of MMP-10, Aβ42 (log2-transformed), P-tau (log2-transformed), and T-tau (log2-transformed) in non-converters (subjective cognitive decline and stable mild cognitive impairment subjects) (A-D), and in converters (progressive mild cognitive impairment subjects) (E-H), further classified in age groups (less than 60 years old, 60-70 years old, 71-80 years old, and over 80 years old). *p < 0 05, **p < 0 01, ***p < 0 001.

### EXAMPLES

### Methods for Assessment

### A. Participants Study

An observational study was performed with the goal of identifying biomarkers of preclinical AD subjects. Participants were derived from two longitudinal memory clinic cohorts, the Spanish Fundaci6 ACE (FACE, subjective cognitive decline [SCD] and mild cognitive impairment [MCI] patients), and the German dementia competence network (DCN, MCI patients). The FACE cohort comprised a) MCI (mild cognitive impairment) patients that were evaluated consecutively at FACE Memory Clinic, and b) healthy individuals with subjective cognitive decline (SCD), participants of Fundació ACE Healthy Brain Initiative (FACEHBI). All participants were assessed with a comprehensive neuropsychological protocol from Fundació ACE (NBACE), and were examined with the Spanish version of Mini-Mental State Examination (MMSE), the Hachinski Ischemia Scale, and Clinical Dementia Rating (CDR). MCI patients fulfilled MCI Petersen's diagnostic criteria, which include subjective memory complaints, decline from normal general cognition, preserved performance in activities of daily living, absence of dementia, and a measurable impairment in one or more cognitive functions, with or without deficit in other cognitive domains (amnestic MCI: single domain, or non-amnestic MCI: multiple domain) and had CDR of 0.5. SCD refers to the perception of memory or other cognitive problems without impairment on standardized cognitive tests. Specifically, individuals with SCD had a CDR of 0, a preserved performance (score ≥ 27) on the MMSE, a score of ≥8 on the Spanish Modified Questionnaire of Memory Failures in Everyday (MFE-30), and a strictly normal performance in the NBACE. MCI follow-up assessments were conducted on an approximately annual basis.

### B. Lumbar punctures and core AD biomarkers measurement

Cerebrospinal fluid (CSF) was obtained via lumbar punctures. After applying subcutaneously local anesthesia (1% mepivacaine), CSF was obtained by puncturing the L3-L4 intervertebral space. The fluid was collected passively in two 10-mL polypropylene tubes (Sarstedt Ref 62.610.018). The first tube was analyzed externally for basic biochemistry analyses (glucose, total proteins, proteinogram, cell types and cell count). The second tube was centrifuged (2000 x g 10 min at 4 °C), and the CSF was divided into 0 325 mL-aliquots in polypropylene tubes (Sarstedt Ref 72.694.007) and stored at -80 °C until further analysis. The delay time between CSF collection and storage was less than 2 hours. On the day of the analysis, one aliquot of CSF was thawed and the levels of Aβ42, P-tau181 and T-tau were measured simultaneously by standard ELISA immunoassays.

In the DCN samples, lumbar punctures from the L3/L4 or L4/L5 intervertebral region were performed. The CSF samples were kept on ice for a maximum of 1 h and then centrifuged for 10 min (2000 g at 4°C). Samples were aliquoted to 0.25 mL and stored in polypropylene tubes at 80 °C until analysis and the levels of Aβ42, P-tau phosphorylated at position 181 (P-tau181) and T-tau were quantified using commercially available ELISA immunoassays.

### C. Targeted Proteomics

Protein concentration was quantified using the validated, highly sensitive and specific ProSeek^{®} multiplex immunoassay, developed by Olink Proteomics (Uppsala, Sweden). Two commercially available ProSeek^{®} Multiplex panels (Inflammation & Neurology) were used to measure the concentrations of 184 proteins in CSF samples from FACE and DCN. Protein quantities are shown as normalized (log2-transformed) protein expression (NPX) values. D. Statistical analysis.

All statistical analyses were conducted using R version 3.6.0. Multiple testing was corrected for False Discovery Rate (FDR) at an FDR-adjusted p-value (i.e. q-value) threshold of 0 05, using the p.adjust function in R. CSF levels of Aβ42, P-tau181 and T-tau were log2-transformed to normalize data.

### E. CSF protein level in relation to diagnosis.

Association of protein expression with diagnosis in FACE was assessed using multiple linear regressions using the Im function in R, where diagnosis was the predictor of interest and CSF protein level the outcome measure. Age and sex were included as covariates. For this analysis, the following diagnosis groups were considered: subjective cognitive decline (SCD), stable mild cognitive impairment (sMCI, i.e. MCI with no progression to ADD during the observational time), and progressive mild cognitive impairment (pMCI, i.e. with progression to ADD at any follow-up exsamination). SCD was employed as the reference category.

### F. Progression from MCI to ADD analysis.

Since the CSF levels of MMP-10 and core AD biomarkers (Aβ42, P-tau181 and T-tau) were measured by different techniques in each cohort, they were z-transformed using the scale function in R to allow comparison of estimates. The impact of protein level on the risk to convert to dementia was assessed using Kaplan-Meier curves (protein concentration divided into tertiles) and Cox proportional-hazard regressions (continuous data) using the survfit and coxph functions, respectively, from the R package survival. Age and sex were included as covariates in Cox regressions.

### G. Risk Stratification.

Subjects from each cohort were classified into the [A/T/(N)] or [A/T/M/N] scheme by converting the CSF levels of Aβ42 (A), P-tau181 (T), MMP-10 (M) and T-tau (N) into binary variables (abnormal, +; normal, -) using proxy cut-off values. Proxy cut-offs were obtained by plotting receiver operating characteristic (ROC) curves (CSF biomarker level as predictor and conversion as outcome) and calculating the Youden index, i.e. the threshold value that provided the best tradeoff between sensitivity and specificity, using the roc and coords functions from the R package pROC. Subjects were then classified into A/T/M/(N) categories as follows: [A-/T-/M-/(N)-], [A-/T-/M+/(N)-], [A+/T-/M-/(N)-], [A+/T-/M+/(N)-], [A+/T+/M-/(N)-], [A+/T+/M+/(N)-], [A+/T+/M-/(N)+], and [A+/T+/M+/(N)+].

### H. Prediction of progression from MCI to ADD.

To compare the ability of a combination of biomarkers to predict conversion to dementia, two different models were built using the values from the training dataset (FACE) and tested in an independent dataset (DCN). I. Correlations and clustering.

Pearson correlation profiles of aging, and CSF levels of MMP-10, log2-transformed Aβ42, log2-transformed P-tau181 and log2-transformed T-tau with ProSeek^{®} Multiplex panels proteins were calculated using the functions cor.test and cor in R. After the correlation test, all values were Fisher z-transformed using the function FisherZ from the R package DescTools. Hierarchical clustering of correlation profiles was performed with the pheatmap function from the R package pheatmap using Pearson-correlation distance.

### RESULTS

### CSF level of MMP-10 Is Increased In both stable and progressive MCI subjects:

In a first step to identify new markers for progression of MCI to dementia and ADD, two commercially available 92-plex panels of proteins were selected based on their involvement in pathogenic processes previously linked to ADD, including neurobiological processes, neurodegenerative diseases, inflammation, and immune response. Thus, 184 proteins were quantified in 365 subjects derived from Fundació ACE (FACE) cohort at different AD clinical stages (subjective cognitive decline [SCD]=50, stable MCI [sMCI]=201, progressive MCI [pMCI]=114). Demographics and clinical characteristics of the cohort are shown in Table 1 below.

| **Table 1. Demographic and clinical characteristics of FACE cohort.** | | | |
|---|---|---|---|
| | **Group** | | |
| **Characteristics** | **SCD** N=50 | **sMCI** N=196 | **pMCI** N=114 |
| Sex (%) | | | |
| *Male* | 50 | 47 5 | 42 1 |
| *Female* | 50 | 52 6 | 57 9 |

| APOE strata (%) | | | |
|---|---|---|---|
| *E2* | 22 | 77 | 7 |
| *E3* | 46 | 63 8 | 47 4 |
| *E4* | 32 | 28 5 | 45 6 |
| Age at lumbar puncture (years, median (IQR)) | 68 (8 75) | 725 (11) | 76 (9) |
| MMSE score (median (IQR)) | 30 (1) | 27 (4) | 24 (5) |
| Time to conversion/last FU (months, median (IQR)) | --- | 14 04 (10 02) | 12 24 (4 8) |

| CSF biomarkers (pg/mL, median (IQR)) | | | |
|---|---|---|---|
| *Aβ₄₂* | 1025 (463 75) | 833 (429 25) | 595 5 (233) |
| *P-tau* | 46 5 (20) | 56 (27 15) | 72 (49 75) |
| *T-tau* | 239 (130 25) | 313 (207 25) | 460 (397 25) |

| | | | |
|---|---|---|---|
| Notations: *APOE* genotypes were classified in E2, E3 and E4 strata as follows: E2, ε2ε2 and ε2ε3; E3, ε3ε3; E4, ε2ε4, ε3ε4, and ε4ε4. SCD, subjective cognitive decline; sMCI, stable mild cognitive impairment; pMCI, progressive mild cognitive impairment; FU, follow-up. | | | |

After excluding 47 proteins due to low detection rate, 137 entered the analysis pipeline. To identify proteins profiling MCI patients at different clinical stages, SCD patients were compared either against sMCI or pMCI groups. This analysis identified ten proteins that were differentially expressed in pMCI compared to SCD, while only five proteins showed a differential expression in the comparison with sMCI. After false discovery rate (FDR) correction, only MMP-10 remained significant in the pMCI vs SCD comparison (p=1 53x10-9, q=2 11x10-7). Interestingly, MMP-10 was also increased in the sMCI group, albeit nominally significant (p=0 007, q=0 49) (Figure 1A-C, Table 2).

| **Table 2. Association of CSF protein levels and diagnosis groups in FACE cohort.** | | | | |
|---|---|---|---|---|
| **Protein** | **Mean difference** | **SE** | ***p* value** | ***q* value** |
| sMCI (Reference category: SCD) | | | | |
| TGFβ1 | -0 21 | 0 06 | 9 14 x 10⁻⁴ | 0 13 |
| MMP-10 | 0 22 | 0 08 | 0 007 | 0 49 |
| CCL-19 | -0 30 | 0 12 | 0 01 | 0 64 |
| CCL-28 | 0 10 | 0 04 | 0 02 | 0 70 |
| CLEC-1β | 0 16 | 0 05 | 0 02 | 0 73 |

| pMCI (Reference category: SCD) | | | | |
|---|---|---|---|---|
| MMP-10 | 0 57 | 0 09 | 1 53 x 10⁻⁹ | 2 11 x 10⁻⁷ |
| MAPT | 0 49 | 0 16 | 0 003 | 0 17 |
| CCL-19 | -0 40 | 0 14 | 0 004 | 0 17 |
| SMOC-2 | 0 26 | 0 09 | 0 006 | 0 20 |
| TGFβ1 | -0 17 | 0 07 | 0 01 | 0 40 |
| CD8α | -0 23 | 0 10 | 0 03 | 0 62 |
| MCP-1 | 0 14 | 0 06 | 0 03 | 0 63 |
| CCL-28 | 0 10 | 0 05 | 0 04 | 0 63 |
| STAMBP | 0 08 | 0 04 | 0 04 | 0 63 |
| CCL-3 | 0 13 | 0 07 | 0 05 | 0 66 |

| | | | | |
|---|---|---|---|---|
| Notations: SE, standard error; p value, nominal p value; q value, false discovery rate-adjusted p value; SCD, subjective cognitive decline; sMCI, stable mild cognitive impairment; pMCI, progressive mild cognitive impairment. | | | | |

Next, the same comparisons were performed as with MMP-10 but using well-established AD biomarkers. It was observed that CSF levels of log2-transformed Aβ42, P-tau181 and T-tau were significantly increased only in the pMCI group (Figure 1D-F). Hence, CSF levels of MMP-10 seemed to increase along the clinical stages in a similar manner as the core AD biomarkers, paralleling the rising of neuropathology. Of note, P-tau181 and T-tau exhibited a strong correlation (Spearman ρ=0 97, p=2 81x10-216, Figure 5), suggesting that similar pathogenic pathways underlie both biomarkers. On the other hand, MMP-10 showed a moderate correlation with P-tau181 (Spearman ρ=0 50, p=1 95x10-24, Figure 5) and T-tau (Spearman ρ=0 54, p=1 57x10-28, Figure 5), and a low negative relationship with Aβ42 (Spearman ρ=-0 23, p=8 74x10-6, Figure 5), strongly suggesting that MMP-10 may serve as an additional AD biomarker that targets pathogenic pathways independent, at least in part, of those tagged by Aβ42, P-tau181, and T-tau (Figure 5).

### CSF level of MMP-10 is associated with progression from MCI to ADD

Based on the observations that the CSF level of MMP-10 increased along the AD clinical staging with the highest level in the pMCI group, to explore whether MMP-10 also showed an association with progression of MCI to ADD. To this end, all FACE MCI samples were divided into tertiles depending on the CSF level of MMP-10, and were analyzed using survival curves. This analysis showed that higher levels of MMP-10 were associated with symptomatic progression (log-rank test, p<1x10-4), similar to P-tau181 (log-rank test, p<1x10-4) and T-tau (log-rank test, p<1x10-4), and opposite to Aβ42 since lower levels of Aβ42 were associated with progression to ADD (log-rank test, p<1x10-4) (Figure 2). In line with these results, Cox regression analysis adjusted for age and sex showed that high levels MMP-10 (HR=1 79, p=3 64x10-9), P-tau181 (HR=1 58, p=9 78x10-6) and T-tau (HR=1 72, p=3 76x10-8), as well as low level of Aβ42 (HR=0 57, p=2 34x10-8) were significantly associated with increased risk (Table 3).

| **Table 3. Association of the CSF levels of MMP-10 and core AD biomarkers with risk of progressing from MCI to ADD in FACE cohort.** | | | |
|---|---|---|---|
| **Protein** | **HR** | **95 % CI** | ***p* value** |
| MMP-10 | 1 79 | 1 47 - 2 17 | 3 64 x 10⁻⁹ |
| log₂ Aβ₄₂ | 0 57 | 0 47 - 0 70 | 2 34 x 10⁻⁸ |
| log₂ P-tau | 1 58 | 1 29 - 1 93 | 9 78 x 10⁻⁶ |
| log₂ T-tau | 1 72 | 1 42 - 2 08 | 3 76 x 10⁻⁸ |

| | | | |
|---|---|---|---|
| Notations: Biomarkers data were z-transformed to allow comparison. All regressions were adjusted for age and sex. HR, Hazard ratio; CI, confidence interval. | | | |

This result prompted to repeat the analysis using an independent sample of MCI subjects with follow-up information, obtained from the longitudinal cohort DCN. The demographic and clinical characteristics of this cohort are shown in Table 4 below.

| **Table 4. Demographic and clinical characteristics of DCN cohort.** | | |
|---|---|---|
| | **Group** | |
| **Characteristics** | **sMCI** N=66 | **pMCI** N=27 |
| Sex (%) | | |
| *Male* | 68 2 | 48.2 |
| *Female* | 31 8 | 51 8 |

| APOE strata (%) | | |
|---|---|---|
| *E2* | 91 | 37 |
| *E3* | 50 | 55 6 |
| *E4* | 40 9 | 40 7 |
| Age at lumbar puncture (years, median (IQR)) | 67 (13 75) | 70 (13 5) |
| MMSE score (median (IQR)) | 28 (3) | 26 (4) |
| Time to conversion/last FU (months, median (IQR)) | 28 05 (20 13) | 26 7 (13 15) |

| CSF biomarkers (pg/mL, median (IQR)) | | |
|---|---|---|
| *Aβ₄₂* | 797 5 (481 75) | 516 (165 5) |
| *P-tau* | 48 5 (29) | 85 (49 5) |
| *T-tau* | 290 5 (219 75) | 536 (356 5) |

| | | |
|---|---|---|
| Notation: APOE genotypes were classified in E2, E3 and E4 strata as follows: E2, ε2ε2 and ε2ε3; E3, ε3ε3; E4, ε2ε4, ε3ε4, and ε4ε4. sMCI, stable mild cognitive impairment; pMCI, progressive mild cognitive impairment; FU, follow-up. | | |

In DCN, the effect of CSF level of MMP-10 were replicated on progression of MCI to ADD previously observed in FACE samples (log-rank test, p<1x10-4). The core AD biomarkers showed the same effect as in FACE, both in survival curves (Aβ42: log-rank test, p=0 002, P-tau181: log-rank test, p =7x10-4, T-tau: log-rank test, p<1x10-4, Figure 6), as well as in Cox regression analysis adjusted for age and sex (Table 5).

| **Table 5. Association of the CSF levels of MMP-10 and core AD biomarkers with risk of progressing from MCI to ADD in DCN cohort.** | | | |
|---|---|---|---|
| **Protein** | **HR** | **95 % CI** | ***p* value** |
| MMP-10 | 2 27 | 1 43 - 3 62 | 5 57 x 10⁻⁴ |
| log₂ Aβ₄₂ | 0 54 | 0 37 - 0 81 | 0002 |
| log₂ P-tau | 1 71 | 1 11 - 2 64 | 0 02 |
| log₂ T-tau | 1 99 | 1 26 - 3 13 | 0003 |

| | | | |
|---|---|---|---|
| Notation: Biomarkers data were z-transformed to allow comparison. All regressions were adjusted for age and sex. HR, Hazard ratio; CI, confidence interval. | | | |

Based on these findings, MMP-10 was explored to check whether it could help improve the capability to predict which MCI patients would progress to ADD. Consequently, a model containing Aβ42, P-tau181, and T-tau together was generated with age, sex, and presence of APOE-ε4 allele, which was compared with an alternative model adding MMP-10. While both models did not differ statistically from each other, they showed a trend towards association (DeLong test, p = 0 12) (Figure 7A-B, Table 6). Similar trend towards association was observed when both predictive models were compared in the independent cohort DCN (DeLong test, p=0 09) (Figure 7C, Table 6). Due to the follow-up interval differences observed for some individuals between DCN and FACE, the analysis was repeated excluding 65 subjects from FACE who had a follow-up of less than 10 months, which is the minimal follow-up interval in DCN. The results of this analysis remained unchanged as compared to the entire sample (Table 6).

| **Table 6. Predictive value of combinations of the CSF levels of MMP-10 and core AD biomarkers in both the training set (FACE cohort) and test set (DCN cohort).** | | | | | | | | | | |
|---|---|---|---|---|---|---|---|---|---|---|
| **Model** | **Training set (FACE)** | | | | | **Test set (DCN)** | | | | |
| | **AUC** | **95 % CI** | **Accuracy** | **Sensitivity** | **Specificity** | **AUC** | **95 % CI** | **Accuracy** | **Sensitivity** | **Specificity** |
| All subjects | | | | | | | | | | |
| *MMP-10, age, sex, APOE-ε4* | 0 76 | 0 70 - 0 81 | 0 72 | 0 68 | 0 75 | 0 74 | 0 63 - 0 84 | 061 | 0 93 | 0 48 |
| *log₂ Aβ₄₂, age, sex, APOE-ε4* | 0 77 | 0 72 - 0 83 | 0 66 | 0 96 | 0 53 | 0 76 | 0 66 - 0 85 | 0 66 | 0 96 | 0 53 |
| *log₂ P-tau, age, sex, APOE-ε4* | 0 72 | 0 66 - 0 78 | 0 66 | 0 82 | 0 56 | 071 | 0 60 - 0 83 | 0 67 | 0 85 | 0 59 |
| *log₂ T-tau, age, sex, APOE-ε4* | 0 74 | 0 68 - 0 80 | 0 67 | 081 | 0 59 | 0 73 | 0 62 - 0 84 | 0 69 | 0 85 | 0 62 |
| *log₂ Aβ₄₂, log₂ P-tau, log₂ T-tau, age, sex, APOE-ε4* | 0 80 | 0 75 - 0 85 | 0 74 | 0 77 | 0 72 | 0 78 | 0 68 - 0 87 | 0 67 | 0 96 | 0 55 |
| *log₂ Aβ₄₂, log₂ P-tau, MMP-10, T-tau, age, sex, APOE-ε4* | 0 82 | 0 77 - 0 86 | 0 78 | 081 | 0 76 | 081 | 0 72 - 0 89 | 0 67 | 1 | 0 56 |
| *age, sex, APOE-ε4* | 0 69 | 0 63 - 0 75 | 0 62 | 0 83 | 0 50 | 0 62 | 0 50 - 0 74 | 0 59 | 0 67 | 0 56 |
| Subjects with FU > 10 months *log₂ Aβ₄₂, log₂ P-tau, log₂ T-tau, age, sex, APOE-ε4* | 0 80 | 0 74 - 0 86 | 0 75 | 0 77 | 0 74 | 0 76 | 0 66 - 0 86 | 0 72 | 0 85 | 0 67 |
| *log₂ Aβ₄₂, log₂ P-tau, MMP-10, T-tau, age, sex, APOE-ε4* | 0 83 | 0 77 - 0 88 | 0 79 | 0 79 | 0 79 | 0 80 | 0 71 - | 0 78 | 0 70 | 0 82 |

| | | | | | | | | | | |
|---|---|---|---|---|---|---|---|---|---|---|
| Notation: The models were trained including all subjects of the training set or including only those subjects with a minimum follow-up of 10 months. Accuracy, sensitivity and specificity were calculated at Youden index. Values of Aβ₄₂, P-tau and T-tau were *z* log₂-transformed, and MMP-10 was z-transformed. FU, follow-up; AUC, area under the curve; CI, confidence interval. | | | | | | | | | | |

### CSF level of MMP-10 provides complementary information to stratify the risk of progressing to ADD In subjects with AD pathological change

The data showed that a model including MMP-10 does not improve importantly (if at all only marginally) its predictive value compared to a model without MMP-10 when applied to the entire MCI population. This is probably because the majority of the MCI patients have, already, abnormal levels of all core AD biomarkers. Thus, they have already paved the way to dementia, and adding any further markers will not improve prediction. For this reason, it was decided to investigate whether MMP-10 would perform better in certain subgroups of the AD continuum. Consequently, MMP-10 was included to the markers already present in the [A/T/(N)] classification. Since MMP-10 is produced by microglia and astroglia due to inflammatory response, and microglial/astroglial pathogenic processes are not covered by the [A/T/(N)] classification, it was hypothesized that assessing the core AD biomarkers together with MMP-10 could provide clinicians with complementary neuroinflammation status to perform a better risk stratification and prognostic evaluation of MCI patients. Thus, the combination of Aβ42, P-tau181, MMP-10, and T-tau in an "[A/T/M/N]" classification was evaluated, with "M" representing the CSF level of MMP-10 as a proxy of microglia/astrocyte-associated dysfunction. To dichotomize the CSF levels of each protein in FACE into normal/abnormal, ROC curves were estimated and the Youden index was used as a proxy for cut-off like previously reported. The proxy cut-off value for Aβ42 was 678 pg/mL (sensitivity=74 6%, specificity=67 3%), 68 pg/mL (sensitivity=57 9%, specificity=73 5%) for P-tau181, 406 pg/mL (sensitivity=59 6%, specificity=74 0%) for T-tau, and 4 23 NPX (sensitivity=72 81%, specificity=60 61%) for MMP-10. The MCI subjects were then stratified into the following groups: [A-/T-/M-/(N)-] (N=85), [A-/T-/M+/(N)-] (N=38), [A+/T/M-/(N)-] (N=26), [A+/T-/M+/(N)-] (N=29), [A+/T+/M-/(N)-] (N=4), [A+/T+/M+/(N)-] (N=1), [A+/T+/M-/(N)+] (N=17) and [A+/T+/M+(N)+] (N=56). The demographic and clinical characteristics of each group are shown in Table 7.

| **Table 7. Demographic and clinical characteristics of each A/T/M/(N) group in FACE cohort.** | | | | | | | | |
|---|---|---|---|---|---|---|---|---|
| **A/T/M/(N) Group** | | | | | | | | |
| **Characteristics** | **A-/T-/M-/(N)-N=85** | **A-/T-/M+/(N)-N=38** | **A+/T-/M-/(N)-N=26** | **A+/T-/M+/(N)-N=29** | **A+/T+/M -/(N)-N=4** | **A+/T+/M +/(N)-N=1** | **A+/T+/M -/(N)+ N=17** | **A+/T+/M+/ (N)+ N=56** |
| Sex (%) | | | | | | | | |
| *Male* | 58 8 | 65 8 | 57 7 | 55 2 | 75 | 100 | 29 4 | 39 3 |
| *Female* | 41 2 | 34 2 | 42 3 | 44 8 | 25 | 0 | 70 6 | 60 7 |

| APOE strata (%) | | | | | | | | |
|---|---|---|---|---|---|---|---|---|
| *E2* | 94 | 15 8 | 11 5 | 34 | 0 | 0 | 0 | 71 |
| *E3* | 75 3 | 73 7 | 42 3 | 48 3 | 25 | 100 | 29 4 | 48 2 |
| *E4* | 153 | 105 | 46 2 | 48 3 | 75 | 0 | 70 6 | 44 6 |
| Age at lumbar puncture (years, median (IQR)) | 70 (11) | 75 5 (9 75) | 73 (5 75) | 77 (11) | 74 5 (1 75) | 80 | 73 5 (13 5) | 76 (8 25) |
| MMSE score (median (IQR)) | 27 (4) | 26 (5) | 26 (3 75) | 24 (5) | 26 5 (3 25) | 21 | 26 (3) | 24 (6) |
| Conversion rate (%) | 59 | 21 1 | 34 7 | 65 5 | 25 | 100 | 58 9 | 66 1 |
| Time to conversion/last FU (months, median (IQR)) | 15 (11 52) | 13 32 (8 97) | 13 86 (12 18) | 12 24 (4 56) | 21 42 (12 15) | 26 9 | 12 24 (6 84) | 12 18 (3 66) |
| CSF biomarkers (pg/mL, median (IQR)) | | | | | | | | |
| *Aβ₄₂* | 970 (259) | 1062 5 (321 5) | 533 (129) | 532 (114) | 414 (109 5) | 604 | 595 (184) | 553 5 (166 25) |
| *P-tau* | 43 (23) | 53 (20 8) | 41 5 (22 5) | 49 (13) | 73 (4 5) | 88 | 100 (28) | 102 5 (44) |
| *T-tau* | 231 (129) | 300 5 (123 5) | 229 5 (98) | 298 (113) | 373 5 (38 75) | 405 | 643 (144) | 695 (341 75) |
| MMP-10 (NPX, median (IQR)) | 381 (0 46) | 451 (0 37) | 381 (0 41) | 4 49 (0 38) | 3 80 (0 08) | 47 | 4 03 (0 33) | 4 74 (0 52) |

| | | | | | | | | |
|---|---|---|---|---|---|---|---|---|
| Notation: *APOE* genotypes were classified in E2, E3 and E4 strata as follows: E2, ε2ε2 and ε2ε3; E3, ε3ε3; E4, ε2ε4, ε3ε4, and ε4ε4. FU, follow-up; NPX, normalized protein expression. | | | | | | | | |

The groups [A+/T+/M-/(N)-] and [A+/T+/M+/(N)-] were excluded from the analysis due to their very small sample size. Following the "amyloid cascade hypothesis", the added value of MMP-10 to disease progression was first explored in those MCI patients showing only amyloid pathology [A+/T-/(N)-]. It was found that MCI A+ patients with an abnormal level of MMP-10 [A+/T-/M+/(N)-] had a higher risk of progression to ADD as compared to those in the M- group ([A+/T/M-/(N)-], median survival time=26 6 months; [A+/T-/M+/(N)-], median survival time=13 4 months; log-rank test, p=0 01) (Figure 4A). Cox regression analysis adjusted for age and sex confirmed the result, with the A+ group posing a ∼ 3-fold higher risk of progressing to ADD when MMP-10 also showed abnormal levels ([A+/T-/M+/(N)-], HR=2 96, p=0 01) (Table 5). Interestingly, it was observed that the sole presence of abnormal CSF level of MMP-10 [A-/T-/M+-/(N)-] increased the risk of progressing to dementia when compared to A-/T-/M-/(N)-, at a level that was similar to that of Aβ42 alone ([A-/T-/M+/(N)-], HR=4 24, p=0 01; [A+/T-/M-/(N)-], HR=5 60, p=0 002) (Figure 4A, Table 4 above). Conversely, no significant differences were observed between MMP-10 groups when P-tau181 and T-tau ([A+/T+/M-/(N)+] vs. [A+/T+/M+/(N)+]) were included in the analysis, both downstream markers of AD pathology (Figure 3B, Table 8).

| **Table 9. Demographic and clinical characteristics of each A/T/M/(N) group** i**n DCN cohort.** | | | | | | | | |
|---|---|---|---|---|---|---|---|---|
| **A/T/M/(N) Group** | | | | | | | | |
| **Characteristics** | **A-/T-/M-/(N)-N=24** | **A-/T-/M+/(N)-N=6** | **A+/T-/M-/(N)-N=20** | **A+/T-/M+/(N)-N=3** | **A+/T+/M-/(N)-N=0** | **A+/T+/M+ /(N)-N=0** | **A+/T+/M-/(N)+ N=4** | **A+/T+/M+/( N)+ N=21** |
| Sex (%) | | | | | | | | |
| *Male* | 62 5 | 83 3 | 60 | 66 7 | - | - | 50 | 52 4 |
| *Female* | 37 5 | 167 | 30 | 33 3 | - | - | 50 | 47 6 |

| APOE strata (%) | | | | | | | | |
|---|---|---|---|---|---|---|---|---|
| *E2* | 167 | 0 | 0 | 0 | - | - | 0 | 48 |
| *E3* | 50 | 83 3 | 55 | 66 7 | - | - | 25 | 38 1 |
| *E4* | 33 3 | 167 | 45 | 33 3 | - | - | 75 | 57 1 |
| Age at lumbar puncture (years, median (IQR)) | 61 (8 25) | 67 (9) | 64 5 (9 25) | 75 (7 5) | - | - | 68 5 (6 25) | 75 (15) |
| MMSE score (median (IQR)) | 29 (2 25) | 27 (3 5) | 28 (1 25) | 29 (2 5) | - | - | 27 5 (1 5) | 26 (3) |
| Conversion rate (%) | 42 | 167 | 15 | 33 3 | - | - | 50 | 71 4 |
| Time to conversion/last FU (months, median (IQR)) | 35 65 (12 93) | 30 6 (18 33) | 25 9 (18 75) | 38 7 (13 95) | - | - | 27 4 (1 78) | 26 (12 6) |

| CSF biomarkers (pg/mL, median (IQR)) | | | | | | | | |
|---|---|---|---|---|---|---|---|---|
| *Aβ₄₂* | 1064 (389 25) | 11175 (167 5) | 547 5 (271 25) | 297 (209 5) | - | - | 455 5 (42) | 582 (158) |
| *P-tau* | 44 (13 8) | 49 5 (23 75) | 45 (19 25) | 37 (14 5) | - | - | 97 5 (19 5) | 100 (42) |
| *T-tau* | 233 5 (73 3) | 319 (161 75) | 267 5 (207 25) | 390 (74 5) | - | - | 555 (238 25) | 727 (3 8) |
| MMP-10 (NPX, median (IQR)) | 3 10 (0 61) | 3 92 (041) | 3 23 (0 48) | 3 72 (0 48) | - | - (0 10) | 3 53 (0 10) | 4 16 (0 48) |

| **Table 8. Association of A/T/M/(N) with the risk of progressing from MCI to ADD In FACE cohort.** | | | |
|---|---|---|---|
| **Group** | **HR** | **95 % CI** | ***p* value** |
| Reference: A-/T-/M-/(N)- | | | |
| A+/*T-*/*M-*/*(N)-* | 5 60 | 1 86 - 16 86 | 0 002 |
| *A-*/*T-*/*M*+/*(N)-* | 4 24 | 1 37 - 13 15 | 0 01 |
| *A*+/*T-*/*M*+/*(N)-* | 14 09 | 5 05 - 39 30 | 4 31 x 10⁻⁷ |
| *A*+/*T*+/*M-*/*(N)*+ | 12 65 | 4 16-38 42 | 7 59 x 10⁻⁶ |
| *A*+/*T*+/*M*+/*(N)*+ | 17 59 | 6 65 - 46 48 | 7 38 x 10⁻⁹ |
| Reference: A+/T-/M-/(N)- | | | |
| *A*+/*T-*/*M*+/*(N)-*Reference: A+/T+/M-/(N)+ | 2 96 | 1 28 - 6 84 | 001 |
| *A*+/*T*+/*M*+/*(N)*+ | 1 31 | 0 61 - 2 83 | 0 39 |

| | | | |
|---|---|---|---|
| Notation: A-/T-/M-/(N)-, A+/T-/M-/(N)-, and A+/T+/M-/(N)+ were used as reference categories. All regressions were adjusted for age and sex. HR, Hazard ratio; CI, confidence interval. | | | |

The findings suggest that the addition of MMP-10 to the markers already present in the current research framework was able to improve the prognostic value for those individuals with amyloid pathology but no tau pathology and/or neurodegeneration. Based on these results, it was decided to stratify the independent DCN cohort with the [A/T/M/N] classification (Table 9).

*Notation: APOE* genotypes were classified in E2, E3 and E4 strata as follows: E2, ε2ε2 and ε2ε3; E3, ε3ε3; E4, ε2ε4, ε3ε4, and ε4ε4. FU, follow-up; NPX, normalized protein expression.

### CSF level of MMP-10 provides a complementary dimension that may be associated with ageing

To gain some insight on the added value of MMP-10 in the [A/T/M/(N)] scheme, all remaining proteins were used in the ProSeek^{®} Multiplex panels and the correlation of these proteins was calculated with age, MMP-10, Aβ42, P-tau181 or T-tau in the same patients to screen for diagnosis-related proteins (SCD, sMCI, and pMCI). Then, a correlation clustering was performed and it was observed that while the correlation profiles corresponding to age and MMP-10 were clustered together, Aβ42, P-tau181 and T-tau formed a different cluster (Figure 4). Finally, our sample was stratified by 10-year age groups and conversion status (SCD plus sMCI vs pMCI). It was noticed that the level of MMP-10 increased steadily with age in SCD and sMCI patients, whereas P-tau181 and T-tau exhibited a plateau effect from 60 years of age on (Figure 8). Interestingly, MMP-10 level in sMCI at an age older than 80 showed a similar level to that in pMCI younger than 60 years of age.

### Observations

It was confirmed that MMP-10 is increased in MCI when compared to SCD patients. It was also proved that a higher MMP-10 level is associated with an increased risk of progressing to AD dementia in both cohorts. Further, MMP-10 has a moderate correlation with P-tau181 and T-tau, and a low negative relationship with Aβ42, indicating that it targets pathogenic independent pathways, at least in part, of those tagged by the core AD biomarkers. Furthermore, it is demonstrated that adding CSF level of MMP-10 to the markers already included in the [A/T/(N)] scheme, with the notation [A/T/M/(N)], improved considerably the prognostic value in MCI patients with abnormal Aβ42, but normal P-tau181 and T-tau. Finally, the data suggest that CSF MMP-10 level might reflect converging ageing and neuroinflammation pathways.

The citations and references used by the inventor are listed below in order to more fully describe the state of the art to which this invention pertains.

The invention has been described in an illustrative manner, and it is to be understood that the terminology, which has been used is intended to be in the nature of words of description rather than of limitation.

Obviously, many modifications and variations of the present invention are possible in light of the above teachings. It is, therefore, to be understood that within the scope of the appended claims, the invention can be practiced otherwise than as specifically described.

### References

- Jack CR, Bennett DA, Blennow K, et al. NIA-AA Research Framework: Toward a biological definition of Alzheimer's disease. Alzheimer's Dement 2018; 14: 535-62.
- Hampel H, O'Bryant SE, Molinuevo JL, et al. Blood-based biomarkers for Alzheimer disease: mapping the road to the clinic. Nat Rev Neurol 2018; 14: 639-52.
- Zetterberg H, Bendlin BB. Biomarkers for Alzheimer's disease-preparing for a new era of disease-modifying therapies. Mol. Psychiatry. 2020; : 1-13.
- Nuttall RK, Silva C, Hader W, et al. Metalloproteinases are enriched in microglia compared with leukocytes and they regulate cytokine levels in activated microglia. Glia 2007. DOI:10.1002/glia.20478.
- Thorns V, Walter GF, Thorns C. Expression of MMP-2, MMP-7, MMP-9, MMP-10 and MMP-11 in Human Astrocytic and Oligodendroglial Gliomas. Anticancer Res 2003.
- Palmqvist S, Hertze J, Minthon L, et al. Comparison of brief cognitive tests and csf biomarkers in predicting alzheimer's disease in mild cognitive impairment: Six-year follow-up study. PLoS One 2012. DOI:10.1371/journal.pone.0038639.
- Jack CR, Hampel HJ, Universities S, Cu M, Petersen RC. A new classification system for AD , independent of cognition A / T / N : An unbiased descriptive classification scheme for Alzheimer disease biomarkers. Neurology 2016*.*
- van Maurik IS, Vos SJ, Bos I, et al. Biomarker-based prognosis for people with mild cognitive impairment (ABIDE): a modelling study. Lancet Neurol 2019; 18: 1034-44.
- Blennow K, Wallin A, Agren H, Spenger C, Siegfried J, Vanmechelen E. tau protein in cerebrospinal fluid - A biochemical marker for axonal degeneration in Alzheimer disease? Mol Chem Neuropathol 1995. DOI:10.1007/BF02815140.
- Sternlicht MD, Werb Z. How matrix metalloproteinases regulate cell behavior. In: Annual Review of Cell and Developmental Biology. Annual Reviews 4139 El Camino Way, P.O. Box 10139, Palo Alto, CA 94303-0139, USA, 2001: 463-516.
- Backstrom JR, Lim GP, Cullen MJ, Tökés ZA. Matrix metalloproteinase-9 (MMP-9) is synthesized in neurons of the human hippocampus and is capable of degrading the amyloid-β peptide (1-40). J Neurosci 1996; 16: 7910-9.
- Yan P, Hu X, Song H, et al. Matrix metalloproteinase-9 degrades amyloid-β fibrils in vitro and compact plaques in situ. J Biol Chem 2006; 281: 24566-74.
- Bjerke M, Zetterberg H, Edman A, Blennow K, Wallin A, Andreasson U. Cerebrospinal fluid matrix metalloproteinases and tissue inhibitor of metalloproteinases in combination with subcortical and cortical biomarkers in vascular dementia and Alzheimer's disease. J Alzheimer's Dis 2011. DOI:10.3233/JAD-2011-110566.
- Craig-Schapiro R, Kuhn M, Xiong C, et al. Multiplexed immunoassay panel identifies novel CSF biomarkers for alzheimer's disease diagnosis and prognosis. PLoS One 2011. DOI:10.1371/journal.pone.0018850.
- Duits FH, Hernandez-Guillamon M, Montaner J, et al. Matrix Metalloproteinases in Alzheimer's Disease and Concurrent Cerebral Microbleeds. J Alzheimer's Dis 2015; 48: 711-20.
- Whelan CD, Mattsson N, Nagle MW, et al. Multiplex proteomics identifies novel CSF and plasma biomarkers of early Alzheimer's disease. Acta Neuropathol Commun 2019. DOI:10.1186/s40478-019-0795-2.
- Lago JC, Puzzi MB. The effect of aging in primary human dermal fibroblasts. PLoS One 2019; 14: e0219165.
- Mora-Gutiérrez JM, Rodríguez JA, Fernández-Seara MA, et al. MMP-10 is Increased in Early Stage Diabetic Kidney Disease and can be Reduced by Renin-Angiotensin System Blockade. Sci Rep 2020; 10: 1-12.
- Montero I, Orbe J, Varo N, et al. C-reactive protein induces matrix metalloproteinase-1 and -10 in human endothelial cells: Implications for clinical and subclinical atherosclerosis. J Am Coll Cardiol 2006; 47: 1369-78.
- Santaella A, Kuiperij HB, Van Rumund A, et al. Inflammation biomarker discovery in Parkinson's disease and atypical parkinsonisms. BMC Neurol 2020; 20: 26.
- Tchkonia T, Zhu Y, Van Deursen J, Campisi J, Kirkland JL. Cellular senescence and the senescent secretory phenotype: Therapeutic opportunities. J. Clin. Invest. 2013; 123: 966-72.
- Lye JJ, Latorre E, Lee BP, et al. Astrocyte senescence may drive alterations in GFAPα, CDKN2A p14 ARF, and TAU3 transcript expression and contribute to cognitive decline. GeroScience 2019; 41: 561-73.
- Heneka MT, McManus RM, Latz E. Inflammasome signalling in brain function and neurodegenerative disease. Nat. Rev. Neurosci. 2018; 19: 610-21.
- Tan FCC, Hutchison ER, Eitan E, Mattson MP. Are there roles for brain cell senescence in aging and neurodegenerative disorders? Biogerontology. 2014; 15: 643-60.
- Liu LR, Liu JC, Bao JS, Bai QQ, Wang GQ. Interaction of Microglia and Astrocytes in the Neurovascular Unit. Front. Immunol. 2020; 11: 1024.
- Hanisch UK, Kettenmann H. Microglia: Active sensor and versatile effector cells in the normal and pathologic brain. Nat. Neurosci. 2007. DOI:10.1038/nn1997.
- De Strooper B, Karran E. The Cellular Phase of Alzheimer's Disease. Cell. 2016; 164: 603-15.
- Sidoryk-Wegrzynowicz M, Wegrzynowicz M, Lee E, Bowman AB, Aschner M. Role of Astrocytes in Brain Function and Disease. Toxicol Pathol 2011; 39: 115-23.
- Beroun A, Mitra S, Michaluk P, Pijet B, Stefaniuk M, Kaczmarek L. MMPs in learning and memory and neuropsychiatric disorders. Cell. Mol. Life Sci. 2019; 76: 3207-28.

## Claims

1. A biomarker for predicting the presence or risk of Alzheimer's disease (AD) in a mammal, or for aiding in the diagnosis of AD and related disorders, comprising metalloproteinase 10 (MMP-10) biomarker, wherein increased levels of the MMP-10 is indicative of the presence, risk, progression or severity of AD.

2. A biomarker of claim 1, wherein said MMP-10 is used in combination with [A/T/(N)] (amyloid/tau pathology/neurodegeneration) classification scheme of AD pathology.

3. The biomarker of claim 1, wherein the biological fluid sample is cerebral spinal fluid (CSF), blood, serum, or plasma.

4. The biomarker of claim 3, wherein the biological fluid sample is cerebral spinal fluid (CSF).

5. The biomarker of claim 1, wherein the mammal is a human.

6. The biomarker of claim 1, wherein the mammal is a mild cognitive impairment patient (MCI).

7. The biomarker of claim 1, wherein said MMP-10 predicts the disease progression at the pre-dementia stage of Alzheimer's disease (AD).

8. The biomarker of claim 1, wherein said MMP-10 predicts the disease progression from mild cognitive impairment (MCI) to overt dementia.

9. The biomarker of claim 1, wherein said MMP-10 predicts the disease progression from mild cognitive impairment (MCI) to Alzheimer's disease dementia (ADD).

10. The biomarker of claim 1, improves the prognostic value in amyloid positive/tau negative mild cognitive impairment (MCI) patients.

11. A method for predicting the presence or risk of Alzheimer's disease (AD) in a mammal, or for aiding in the diagnosis of AD and related disorders, a method comprising determining the amount of metalloproteinase 10 (MMP-10) biomarker; wherein increased levels of the MMP-10 is indicative of the presence, risk, progression or severity of AD.

12. A method of claim 11, wherein said metalloproteinase 10 (MMP-10) is used in combination with [A/T/(N)] (amyloid/tau pathology/neurodegeneration) classification scheme of AD pathology biomarker.

13. The method of claim 11 or 12, wherein the biomarkers are obtained from a biological fluid sample.

14. The method of claim 13, wherein the biological fluid sample is cerebral spinal fluid (CSF), blood, serum, or plasma.

15. The method of claim 14, wherein the biological fluid sample is cerebral spinal fluid (CSF).

16. The method of claim 11, wherein the mammal is a human.

17. The method of claim 11, wherein the mammal is a mild cognitive impairment patient (MCI).

18. The method of claim 11, wherein the mammal is an amyloid positive/tau negative mild cognitive impairment (MCI) patient.

19. The method of claim 11, wherein said MMP-10 predicts the disease progression at the pre-dementia stage of Alzheimer's disease (AD).

20. The method of claim 11, wherein said MMP-10 predicts the disease progression from mild cognitive impairment (MCI) to overt dementia.

21. The method of claim 11, wherein said MMP-10 predicts the disease progression from mild cognitive impairment (MCI) to Alzheimer's disease dementia (ADD).

22. A kit comprising the matrix metalloproteinase 10 (MMP-10), wherein the kit is used to determine the presence or risk of AD in a mammal, or for aiding in the diagnosis and AD and related disorders.

23. The kit of claim 22, wherein said matrix metalloproteinase 10 (MMP-10) is used in combination with [A/T/(N)] (amyloid/tau pathology/neurodegeneration) classification scheme of AD pathology biomarker.

24. The kit of claim 22 or 23, wherein the biomarkers are obtained from a biological fluid sample.

25. The kit of claim 24, wherein the biological fluid sample is cerebral spinal fluid (CSF), blood, serum, or plasma.

26. The kit of claim 1, wherein the mammal is a mild cognitive impairment patient (MCI).

27. The kit of claim 22, wherein said MMP-10 predicts the disease progression at the pre-dementia stage of Alzheimer's disease (AD).

28. The kit of claim 22, wherein said MMP-10 predicts the disease progression from mild cognitive impairment (MCI) to overt dementia.

29. The kit of claim 22, wherein said MMP-10 predicts the disease progression from mild cognitive impairment (MCI) to Alzheimer's disease dementia (ADD).
